Europäisches Patentamt

European Patent Office

Office européen des brevets

(19)

(11) **EP 0 977 902 B1**

(12) **EUROPEAN PATENT SPECIFICATION**

(45) Date of publication and mention
of the grant of the patent:
**07.07.2004  Bulletin 2004/28**

(21) Application number: **97919618.5**

(22) Date of filing: **25.04.1997**

(51) Int Cl.[7]: **C22F 1/10**, C22F 1/18
// C22K1:00

(86) International application number:
**PCT/IL1997/000134**

(87) International publication number:
**WO 1998/049363 (05.11.1998 Gazette 1998/44)**

(54) **MANUFACTURE OF TWO-WAY SHAPE MEMORY DEVICES**

HERSTELLUNG VON ZWEIWEGFORMGEDÄCHTNISTEILEN

FABRICATION DE DISPOSITIFS A MEMOIRE DE FORME BIDIRECTIONNEL

(84) Designated Contracting States:
**AT BE CH DE DK ES FI FR GB GR IE IT LI LU MC
NL PT SE**

(43) Date of publication of application:
**09.02.2000  Bulletin 2000/06**

(73) Proprietor: **Litana Ltd.**
**58824 Holon (IL)**

(72) Inventors:
• **FLOMENBLIT, Josef**
**58824 Holon (IL)**

• **BUDIGINA, Nathaly**
**58824 Holon (IL)**

(74) Representative: **Meissner, Bolte & Partner
Anwaltssozietät GbR
Postfach 86 06 24
81633 München (DE)**

(56) References cited:
**WO-A-89/10421          US-A- 4 283 233**

## Description

## FIELD OF THE INVENTION

The present invention generally relates to shape memory alloys (SMA) i.e. alloys which can switch from one shape to another, *"memorized"* state upon a change in temperature. More specifically, the present invention relates to an SMA which is nickel-titanium based, also known as Nitinol.

## BACKGROUND OF THE INVENTION

Various metal alloys possess the ability to change their shape as a result of a change in temperature. Such SMA can undergo a reversible transformation from a martensitic state, in which the material is relatively soft and deformable, to an austenitic state in which the material possesses super elastic properties and is relatively firm. The transformation from the martensitic state to the austenitic state will be referred to herein as the *"austenitic transformation "*, and the other transformation, from the austenitic state to the martensitic state, will be referred to herein as the *"martensitic transformation"*. The austenitic transformation occurs over a range of temperature which is higher than the range of temperatures in which the reverse transformation occurs. This means, that once transformed to an austenitic state, an SMA will remain in that state even when cooled to a temperature below that in which the austenitic transformation began, as long as the temperature is above that in which the martensitic transformation begins.

A particular class of SMAs are alloys of nickel and titanium - NiTi alloys. NiTi alloys have found a variety of uses in medical as well as other fields. Medical uses of SMA's, particularly an NiTi-based alloy has been described in U.S. Patents 4,665,906, 5,067,957, European Patent Application 143,580, U.S. Patent 4,820,298 and many others.

For medical uses, it is usually desired that the alloy will undergo an austenitic transformation over a narrow, well defined range. For example, a vascular stent of the two-way SMA type, such as that described in European Patent Application, Publication No. 625153, is typically deployed in the body while being in the martensitic state at body temperature, and then after heating, it transforms into the austenitic state, and then remains in the austenitic state when cooled to the body temperature. It can be appreciated that if excessive heating to transform the SMA from the martensitic to the austenitic state is required, this can be damaging to the surrounding tissue and is thus undesirable. Thus, it would ideally be desired that the austenitic transformation will begin at a temperature several degrees above body temperature and will be over a temperature range which will not cause tissue damage owing to the excessive heating.

WO 89 10421-A discloses a process for controlling the physical and mechanical properties of a nickel-titanium alloy exhibiting shape memory effect, said process comprising the following steps:

- annealing at a temperature of from 300 to 950°C for 5 minutes to two hours
- cold-working in the range of 6 to 60%
- forming to a desired shape of configuration
- heat treating at a selected memory imparting temperature of from 400 to 600°C,
- and cooling.

GENERAL DESCRIPTION OF THE INVENTION

It is an object of the present invention to provide a process fcr the treatment of a NiTi-based alloy to obtain an alloy with a shape memory effect (SME), having reversibly adjustable characteristic transformation temperatures.

It is more specifically an object of the invention to provide such a process to obtain a two-way SME which does not require a multi-cycle "*training*" to yield a two-way SME.

It is still further an object of the invention to provide a process to obtain a two-way SME, with a narrow range of temperatures where the austenitic transformation occurs.

The process of the invention has two aspects. By one aspect, to be referred to herein at times as the "*said first aspect*", the process yields an alloy with a direction of the austenitic and the martensitic transformations

The document US-A-4 283 233 discloses a process for changing the shape transition temperature range of a nickel-titanium alloy comprising the following steps :

- optionally hot-swagging,
- cold-working a minimum of 15% such that the permanent shape is obtained,
- annealing at a temperature between 400 and 600°C for a time at least longer than 5 minutes after the object has reached the annealing temperature,
- cooling down,
- and forming by conventional techniques into another shape with less than 7% deformation.

dictated by the direction of a conditioning transformation in the martensitic state. In accordance with another aspect of the invention, to be referred to herein at times as the "*said second aspect*", the process yields an alloy with a direction of a martensitic or austenitic transformation which is indpendent on the deformation introduced in the martensitic state.

In the following description and claims, the term "*NiTi alloy*" will be used to denote an alloy comprising primarily nickel and titanium atoms but may contain also trace amounts of other metals). A NiTi alloy has typically the following empiric formula:

$$Ni_1\ Ti_m\ A_n$$

Wherein

A represents Cu, Fe, Cr or V

l, m, and n representing the proportions of the metal atoms within the alloy, the value of l, m and n being about as follows:

l = 0.5
m = 0.5-n
n = 0.003 to 0.02

In accordance with the invention which is defined in claim 1, there is provided a process for treating a raw NiTi alloy having an initial form to obtain an alloy with a final form in which it exhibits a two-way shape memory effect (SME) whereby it has an austenitic and a martensitic memory state with associated austenitic and martensitic shapes, respectively, the process comprising the steps of:

(a) testing the raw NiTi alloy so as to obtain an estimate of the alloy's internal structure, by measuring the difference between $A_s$ and $A_f$;
(b) subjecting the raw NiTi alloy to a fist heat treatment based on results obtained in (a) so as to yield alloys with an initial internal structure condition having essentially equal random dislocation density wherein the first heat treatment is defined such that:

- where the difference is less than 7°C, heat treating the alloy to a temperature of 450-500° for 0.5-1.0 hours;

- where the difference is more than 7°C, heat treating the alloy to a temperature of 510-550° for 1.0-2.5 hours;

(c) subjecting the alloy to thermo-mechanical treatment (TMT) at a temperature above 0.3 Tm where Tm is a melting temperature of the alloy in Kelvin, comprising plastic deformation of the alloy with simultaneous heating (e.g. by warm rolling or warm drawing) to yield, during dynamic ageing process (ageing while depressing), a polygonal sub-grain dislocation structure decorated by precipitation;
(d) if the deformation in step (c) did not yield the final form, subjecting the alloy to an intermediate heat treatment to conclude one cycle of sub-grain dislocation structure formation;
(e) repeating steps (c) and (d) until yielding said final form; and
(f) subjecting the alloy to a final heat and memorizing treatment, to yield two memory states consisting of an austenitic state, in which said alloy has an austenitic form and a martensitic state, the treatment comprising:

(i) forming the alloy into said austenitic form; subjecting the alloy to a polygonization treatment in the range from 450 to 550°C to yield arrangement of random dislocation, then to solution treatments to release non-arranged dislocation from precipitation and provide for their rearrangement and then to an aging treatment; and deforming the alloy to assume a conditioning form with a higher degree of deformation than said martensitic form and heating to obtain the two memory states; or

(ii) forming the alloy into a conditioning form with a higher degree of deformation than the martensitic form; subjecting said alloy to heat treatment, then to polygonization and solution treatment in the range from 600 to 800°C and then optionally to an aging treatment; forming the alloy into said austenitic form and subjecting the alloy to a memorizing heat treatment to an aging treatment.

The TMT, while it may in some instance be performed in a single step, occasionally it is necessary to do it in a few steps if the total strain value could exceed a critical value which may give rise to formation of precracks (crack nucleation) in the alloy. The TMT is performed while warming the alloy typically to a temperature of about 0.3 - 0.6 Tm (Tm = melting temperature in °K).

In accordance with one embodiment of the invention, the process comprising the steps of:

(a) heating a sample of the raw NiTi alloy, to a temperature of about 450-550°C for about 0.5-2.5 hours, and then testing the sample for temperature difference between $A_s$ and $A_f$;
(b) subjecting the raw NiTi alloy to a first heat treatment based on the $A_f$-$A_s$ difference obtained in step (a), as follows:

- where the difference is less than about 7°C, heat treating the alloy to a temperature of about 450-500°C for about 0.5-1.0 hours;
- where the difference is more than about 7°C, heat treating the alloy to a temperature of about 510-550°C for about 1.0-2.5 hours;

(c) subjecting the alloy to thermo-mechanical treatment, comprising plastic deforming the alloy at a strain rate, of less than 5 $sec^{-1}$, with simultaneous internal heating of a portion of the alloy where the deformation occurs to a temperature of about 250-550°C, the deformation of this step being less than 55%, preferably less than 40%;

(d) if the deformation in step (c) did not yield the final form, subjecting the alloy to an intermediate heat treatment at a temperature of about 500-550°C, for about 0.5-2 hours, and then repeating step (c); and

(e) subjecting the alloy to a final heat treatment and to a memorizing treatment.

The particulars of the final heat treatment and the memorizing treatment, are different in said first aspect and in said second aspect. In accordance with said first aspect, this treatment comprises:

(i) forming the alloy into the form to be assumed by it in the austenitic state,

(ii) subjecting the alloy to a polygonization heat treatment to yield arrangement of random dislocation, then to solution treatment to release non arranged dislocation from precipitation and provide for their rearrangement and then to an ageing treatment;

(iii) deforming the alloy to assume a conditioning form and treating it to memorize said austenitic state, which is the state into which it was formed under (i) above, and a martensitic state, in which the alloy has a martensitic form with an intermediate degree of deformation between the austenitic form and the conditioning form.

Preferably, steps (ii) and (iii) of said first aspect, comprise:

(ii) subjecting the alloy to a polygonization heat treatment at about 450-550°C for about 0.5-1.5 hours, then to solution treatment at about 600-800°C for about 2-50 mins., and then to an ageing treatment at about 350-500° for about 0.15-2.5 hours, and

(iii) deforming the alloy to assume a conditioning form, the deformation being less than about 15%, and preferably less than 7%, and being performed at a temperature T, which meets the following formula

$$T < M_s + 30°C$$

wherein $M_s$ is a temperature where the martensitic transformation begins, and then heating the alloy to a temperature of or above that in which the austenitic transformation of the alloy ends.

It should be pointed out that although a single cycle of deformation in step (iii) above is usually sufficient, it may at times be desired to repeat this cycle one or more times.

In accordance with said second aspect, the final heat and memorizing treatment comprises:

(i) forming the alloy into a form other than the form to be assumed by it in the austenitic state,

(ii) subjecting the alloy first to heat treatment, then to polygonization and solution treatment and then optionally to ageing treatment;

(iii) forming the alloy into a form to be assumed by it in the austenitic state,

(iv) subjecting the alloy to a memorizing heat treatment and to an ageing treatment;

whereby the alloy is conditioned to memorize an austenitic state in which it has an austenitic form assumed by it in (iii) above, and a martensitic state, wherein it has a martensitic form being a form with an intermediate degree of deformation between the form in which the alloy was formed in (i) above and the austenitic form.

By a preferred embodiment of said aspect, steps (ii) and (iv), comprise:

(ii) subjecting the alloy to a heat treatment at about 450-500°C for about 0.5-2 hours, then subjecting the alloy to polygonization and solution treatment at about 600-800°C for about 2-50 mins., and then subjecting the alloy to aging treatment at about 350-500°C for about 0-2 hours,

(iv) subjecting the alloy to a memorizing heat treatment at about 500-600°C for more than about 10 mins., and then subjecting the alloy to ageing treatment at about 350-500°C for about 0.15-2.5 hours.

Following the treatment in accordance with both said first and said second embodiments, $A_f$ will be between about 10 to about 60°C. In order to increase $A_f$ and $A_s$, the alloy may then be subjected to ageing heat treatment at a temperature of about 350-500°C. In order to decrease $A_f$ and $A_s$, the alloy can then be subjected to a solution treatment at a temperature of about 510 to about 800°C.

By differential ageing or solution treatment in different portions the alloy will have different temperatures of austenitic transformation. This is at times desired, for example, in the case of a medical stent, to have portions thereof with different transition temperatures of austenitic transformation and/or martensitic transformation.

By the above process, SMAs for a variety of applications may be prepared. Examples are medical devices, e.g. various orthopaedic devices, tooth rooth implants, medical stents, intrauterine implants, as well as non-medical devices, e.g. tube joints. A process for the preparation of such medical devices, as well as devices prepared by such process also form an aspect of the invention.

## BRIEF DESCRIPTION OF THE DRAWINGS

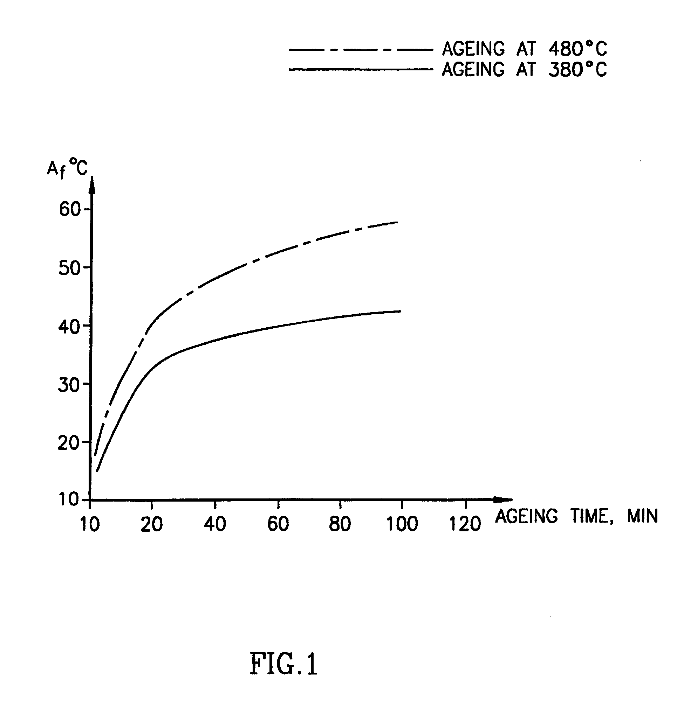

**Fig. 1** in the drawings shows the relation between $A_f$ and the ageing time, in different ageing temperatures.

## DETAILED DESCRIPTION OF THE INVENTION

The temperature range over which the austenitic transformation takes place, is critical in a variety of medical applications. A specific case in point are medical stents such as those made of a two-way shape memory alloy (SMA) described in European Patent Application No. 626153. Such a shape memory (SM) device, is deployed in a tubular organ at body temperature, and then heated so as to allow the occurrence of the austenitic transformation. Once heated, it remains in the austenitic state in body temperature and supports the wall of the tubular organ. Such SM devices are designed so that the beginning of the austenitic transformation will occur at a temperature at or above 40°C. It will however be appreciated, that the range of temperature over which the austenitic transformation occurs should desirably be narrow since excessive heating where the temperature range is large, can cause tissue damage. Furthermore, a narrow temperature range will generally ensure also a more rapid transition from the martensitic to the austenitic states.

In the following description the invention will be described at times with particular reference to its application for the preparation of medical SM devices with a narrow range of austenitic transformation. It will however, be appreciated, that the invention is not limited thereto and the application of the invention to the preparation of medical stents is exemplary only. In accordance with the present invention, a raw NiTi alloy, which is typically provided by manufacturers in the form of a wire or a rod is first tested for the difference between $A_s$ and $A_f$. For this purpose a small sample of the material is taken. Based on the $A_s$-$A_f$ difference, the alloy, e.g. the wire or the rod, is then subjected to a first heat treatment.

Following the first heat treatment, the alloy is subjected to a thermo-mechanical treatment (TMT) where the alloy is simultaneously heated and subjected to a mechanical deformation. In the case of a process intended for the manufacture of a medical SM device, the mechanical deformation typically involves changing the form of the alloy, from an initial form of a wire or rod, to that of a ribbon, band, etc; or alternatively, changing the wire or rod into a wire or rod of a smaller diameter. In order to retain the shape memory effect (SME) of the alloy, the total degree of deformation during the TMT, should be less than 55%, preferably less than 40%. Where the total required eventual deformation is more than 55%, the TMT is performed in two steps with an intermediate heat treatment.

The thermo-mechanical treatment may, for example, be: warm rolling where the alloy is processed to be used as a medical stent; warm drawing where the alloy is processed to be used as an orthopaedic tooth root implant; etc. In warm rolling or drawing the alloy is typically heated to a temperature of about 0.3-0.6 Tm (Tm being the melting temperature in °K). The heating of the deforming portion must be by electro-stimulation, e.g. at a current density of about 500-2000 A/cm$^2$. A big advantage of such a treatment is that in addition to causing a mechanical deformation, it leads also to heating of the pre-cracks with a high dislocation density owing to the relatively high electrical resistance at such pre-cracks which gives rise to a selective overheating at such points and heating of the pre-cracks. Moreover, electro-stimulated warm TMT at the above current density accelerates dislocation reaction, which results in a perfect dislocation subgrain structure formation. Furthermore, the heating electrical current gives rise to a dynamic ageing process with a second phase precipitation on the walls of the subgrain dislocation cells. This structure provides for a very narrow thermal interval of austenitic transformation $A_f$-$A_s$ for the shape memory alloy, and for a variety of other advantageous properties to be explained below.

In an electrically stimulated warm rolling, where the current density decreases below 500 A/cm$^2$, or the strain rate of the deformation is above about 5 sec$^{-1}$, there is an increase of the random dislocation density which will decrease the degree of perfection of the subgrain structure. For a narrow $A_f$-$A_s$ interval, a subgrain structure as perfect as possible is required. Accordingly, with an increase in the random dislocation density there is an increase in the $A_f$-$A_s$ interval. For example, where the current density is about 400 A/cm$^2$, or where strain rate is about 8 sec$^{-1}$, the $A_f$-$A_s$ interval after final heat treatment will be about 10-12°C. Furthermore, increasing of the current density to above about 2000 A/cm$^2$, leads to a recrystallization process, that prevents formation of the necessary subgrain cells with precipitation on the cell walls.

The memorizing treatment involves a conditioning step in which microscopic changes within the alloy condition it to *"memorize"* the two forms which the alloy assumes during its use, that in the martensitic state (*"martensitic form"*) and that in the austenitic state (*"austenitic form"*).

In accordance with said first aspect, the alloy is formed into a shape to be assumed by it in the austenitic state, e.g. in the case of a stent this involves winding on a mandrel having a diameter of a stent in the austenitic state. The alloy is then typically placed in a vacuum or inert atmosphere furnace, in which it is first subjected to a memorizing and internal structure polygonization treatment, at a temperature of about 450-550°C for about 0.5-1.5 hours, and then heated to about 600-800°C for about 2-50 mins. During this latter heating, the alloy undergoes a solution treatment with re-arrangement of dislocations which are freed after solution treatment. Subsequently, the alloy is subjected to a final aging treatment at a temperature of about 350-500°C for about 0.15-2.5 hours.

The result of the above treatment is a subgrain structure which imparts the alloy with several features. For one, the temperature of the austenitic transformation, $A_f$, can be adjusted within a range of 10-60°C with a very narrow interval of $A_f$-$A_s$ of about 1-5°C.

In case it is desired to decrease $A_f$, the alloy may be subjected to a solution treatment at a temperature of about 510-800°C. In order to achieve a desired $A_f$, both the temperature as well as the ageing time can be controlled. For example, where the Nitinol alloy has after the final heat treatment an $A_s$ of about 45°C and an $A_f$ of about 48°C, after a solution treatment at 640°C for about 5 mins., the $A_s$ and $A_f$ decrease to about 23°C and 27°C, respectively; following solution treatment at 640°C for 10 mins., $A_s$ and $A_f$ decrease to about 11°C and 15°C, respectively.

In order to increase $A_f$, the alloy is subjected to an ageing heat treatment at a temperature of about 350-500°C. Here again, in order to achieve a desired $A_f$, both the temperature as well as the ageing time can be controlled. This is demonstrated, for example, in Fig. 1 which gives the relation between the ageing time at two different temperatures (380°C and 480°C) and the resulting $A_f$, following a solution treatment at 640°C for 20 mins. As can be seen, for example, ageing treatment at 380°C for about 100 mins. yields an $A_f$ of about 40°C, with the same $A_f$ being reached with an ageing treatment at 480°C of about 40 mins. Ageing at a temperature of about 450°C for about 80 mins. will yield an $A_s$ of about 46°C and an $A_f$ of about 49°C (not shown in Fig. 1).

A unique feature of the process of the invention is the fact that the two-way SME is induced by only one cycle of deformation. In the case of said first aspect of the invention, this may bc achieved by deforming the alloy into a conditioning form at a temperature of $T < M_s + 30°C$ followed by heating to a temperature at or above the $A_f$ of the alloy. The deformation should be less than 15% and preferably less than 7%. A deformation above 15% will effect the internal structure of the material and yield a total or partial loss of the memory form of the austenitic state. A deformation between 7% and 15% will have only such a partial harming effect. The martensitic memory form which the alloy assumes after the above conditioning step, is an intermediate form between the austenitic memory form and the conditioning form. The direction of the two-way SME following such memorizing treatment, coincides with the direction in the martensitic state deformation. For example, whcre a deformation in the martensitic state involves a decrease in diameter, the diameter of the alloy in the martensitic state will be less than that of the austenitic state, and *vice versa*.

Generally, the process in accordance with said first aspect allows a reversible adjustment of the characteristic transformation temperatures as well as the direction of the two-way SME at the final stage of manufacture.

A final memorizing treatment in accordance with the second aspect of the invention, gives rise to a two-way SME without the need for a final deformation to induce the two-way SME. This effect is not determined when indirect SME occurs. The second aspect is useful, for example, for the manufacture of a stent with a two-way SME, and the description below will refer to this specific embodiment. The NiTi ribbon or wire is wound on a mandrel having a diameter equal to $2R_1$ constrained and placed into a vacuum furnace, at a temperature of about 450-550°C for about 0.5-2.0 hours, so that internal structure normalization and textural formation takes place. Similarly as above, the alloy is then subjected to solution treatment and structure improvement at a temperature of 600-800°C for 2-50 min., and then to an aging treatment at 350-500°C for 0 - 2.5 hours. The ribbon or wire is then rewound on a mandrel with a diameter $2R_2$, which is the diameter to be assumed by the stent in the austenitic state and then subjected to a memorizing treatment at 500-600°C for more than 10 min. and aging treatment at temperature of 350-500°C for 0.15-2.5 hours. If the strain of this treatment $\varepsilon_{treat} = \frac{1}{2}w(1/R_2 - 1/R_1) < 0$ (w being the thickness in case of a ribbon and the diameter in case of a wire) the corresponding strain of the two-way SME during cooling $\varepsilon_{tw} = \frac{1}{2}w(1/R_{tw} - 1/R_2) > 0$ ($2R_{tw}$ being the diameter of the stent when assuming its martensitic state) and *vice versa*. As a result of this treatment, there is a very narrow temperature range in which the austenitic transformation takes place, $A_f - A_s = 1-5°C$, with a possibility to change $A_f$ between 10 and 60°C, similarly, as above.

The two-way SME in cooling may either coincide or oppose the direction of the deformation in the martensitic state. In case $R_2$ is larger than $R_1$, and $R_{tw}$ will be smaller than $R_2$, the device shrinks when it is cooled. In case $R_2$ is less than 0, i.e. a reverse bending, and $R_2$ is larger than $R_{tw}$, the device will expand when cooled.

Finally, another result of the process of the present invention is a high resistance of the formed alloy, to pitting corrosion and hydrogen embrittlement which may occur in the biological media with their relatively high chlorine ion content.

The invention will now be illustrated further by several specific examples.

## Example 1 - Preparation of a Biliary Stent

The starting material was a super-elastic NiTi wire, with a diameter of 1.5 mm. The Ti and Ni content of the alloy was 50 to 50.8 at % (at % = % atoms out of the total number of atoms in the alloy) and 49.1 at %, respectively. A sample of the wire was treated at a temperature of 500°C for 1.5 hours and the temperature interval $A_f$-$A_s$ was determined and was found to be 15°C.

The wire was then subjected to a first heat treatment at 550°C for two hours, and then to a thermo-mechanical electro-simulated treatment, with the current density being 900 A/cm$^2$ and the strain rate being 0.3 sec$^{-1}$. The TMT was repeated three times with two intermediate heat treatments at 500°C for one hour each. The ribbon thickness was eventually reduced to 0.25 mm.

The ribbon was then wound and constrained on a mandrel having a diameter of 8 mm, and placed into a vacuum furnace and heated to 500°C for 0.6 hours, and then subjected to a solution treatment at 650°C for 30 mins. This was followed by an aging treatment at 400°C for 1 hour.

The spiral coiled stent which was obtained had an $A_s$ of 40°C and an $A_f$ of 43°C.

The stent was then wound on a 3 mm. diameter mandrel at a temperature of 25°C and heated to above 43°C for shape recovery. Thus, a stent with a two-way SME was obtained, having an austenitic memory form in which its diameter was 8 mm, a martensitic memory form to which it shrank when cooled below 25°C in which it had a diameter of 7.3 mm.

In order to install the stent, *in situ* within the body, it is wound on a catheter, and then inserted into the desired place within the bile duct. The stent is then activated by raising its temperature to more than 43°C. To remove the stent it has to be cooled below 25°C and after shrinking it can be pulled away.

### Example 2 - Esophageal Stent

A stent was prepared from the same TiNi wire as used in Example 1. The wire was subjected to a first heat treatment and then to a TMT, similarly as described in Example 1, the difference being that the final thickness of the wire which was obtained was 0.28 mm.

The ribbon was then wound on a mandrel having a diameter of 70 mm, was constrained and then heated to 500°C for 1 hour and then to a solution treatment at 650°C for 20 mins. The ribbon was then wound on a mandrel having a diameter of 16 mm., was constrained and subjected to a memorizing treatment at 520°C for 30 mins., and then to aging treatment at 400°C for 2 hours. The stent which was obtained after this procedure had the following parameters: $A_s$ = 42°C; $A_f$ = 45°C; temperature of martensitic transformation being 27°C, with the stent expanding when cooling from a diameter of 16 mm. which it had in the austenitic state to a diameter of 18 mm. in the martensitic state.

For deployment, the stent is wound on a catheter with a diameter of 5 mm, inserted into the desired place within the esophageal tract and is activated by heating above 45°C. When the stent is cooled, it expands which prevents the stent from falling into the stomach.

### Example 3 - Esophageal Stent

A stent was prepared in a similar manner as that of Example 2, with the difference being that the ribbon was wound on a mandrel having a diameter of 5 mm. and after heat treatment was rewound on the mandrel with the opposite direction. After heat treatment, similarly as in Example 2, the stent expands when cooled from a diameter of 16 mm. to a diameter of 25 mm.

### Example 4 - Shape Memory Force Element for Orthopedic Compression Screw

Starting material was 1.5 mm diameter NiTi wire (the composition of the alloy was 50.5 at % Ni and 49.5 at % Ti). The wire was subjected to a first heat treatment and then to a TMT, similarly as described in Example 1 (however using warm drawing instead of warm rolling).

The wire was then heat treated in straight constrained condition under a temperature of 500°C for 0.5 hours and then subjected to a solution treatment at 650°C for 20 mins. The wire was then released and subjected to a memorizing treatment at 520°C for 30 mins., which was followed by an ageing treatment at 450°C for 1 hour. After wire elongation from 20 to 21 mm, shape memory effect was obtained with $A_s$ = 39°C and $A_f$ = 41°C and after cooling up to 25°C two way shape memory effect occurs just after heat treatment (without training) and increases after the training process (stretching-heating).

### Example 5 - Shape Memory Medical Staples

Starting material and treatment was similar to that of Example 4. The final diameter which was achieved (by warm drawing) was 0.25 mm. The wire was constrained in the necessary shape and heat treated after TMT at 520°C for 0.5 hours, solution treatment at 680°C for 10 mins. and ageing at 450°C for 1.5 hours.

After staple bending, shape memory effect was obtained with $A_s$ = 42°C and $A_f$ = 45°C.

### Example 6 - Tooth Root Implants

The starting material was a super-elastic Nitinol (50.8 at % Ni) rod with diameter of 10 mm. The rod was subjected to a first heat treatment at 550°C for 2 hours and then to a TMT - drawing at 500°C, with a strain rate of 0.5 sec$^{-1}$. The TMT was repeated 2 times with intermediate heat treatment at 500°C for 1 hour. The rod had a final diameter of 6.0 mm.

The rod was machined into a shape of a tooth root implant with 6 force segments (legs) for anchoring into the jaw bone, The leg's length was 3,4 and 5mm for different implants. The implant was then subjected to polgonization heat treatment at 500°C, for 1 hours, then the implant's lets were distorted on the mandrel, following which the implant was subjected to heat treatment at 650°C, for 30 min. and to aging at 480°C for 1.5 hours. Then the implant's legs were forced together with a conic cup, (from distorted diameter 5.0mm to closed conditions with diameter 3.0mm). While heating the implant, it causes the legs to open at temperatures: $A_s$=38 and $A_f$=42 C, that yields very gentle pressure on the jaw bone and very safe implant activation. A single cycle of straightening of the implant's legs and subsequent heating induces two way SME in the direction of joining of the legs with cooling, which feature is useful for removing the implant.

### Example 7 - Two way SM tube coupling with narrow $A_s$-$A_f$ interval

A 10 mm NiTi rod identical to that which served as the starting material in Example 6 was treated in the same manner as described in Example 6 to yield a rod with a diameter of 6 mm. This rod was then machined into the shape of the hollow cylinder with an internal conditioning diameter (ID) of 4.4 mm. Then the cylinder was subjected to poligonization and solution heat treatment: 500°C for 1 hour and then of 680°C for 20 min. It was then cooled, expended on a mandrel with a diameter of 4.5 mm and subjected to memorizing and aging heat treatment: 530°C for 30 min. and 430°C for 40 min. The tube coupling was then cooled and expanded on the mandrel to yield an internal diameter of 4.75 mm.

The coupling joins tubes after heating ($A_s$=15°C, $A_f$= 18°C) and it has two way SME in direction of reduction of ID. Thus, even with cooling it keeps pressure on the joined tubes. In comparison, conventional couplings where the two way SME is induced during installation (expansion and heating) in direction of expansion, cooling results in loosening of the coupling.

### Claims

1. A process for treating a raw NiTi alloy having an initial form to obtain an alloy with a final form in which is exhibits a two-way shape memory effect (SME) whereby it has an austenitic and a martensitic memory state with associated austenitic and martensitic shapes, respectively, the process combining the steps of:

   (a) testing the raw NiTi-based alloy so as to obtain an estimate of the alloy's internal structure by measuring the difference between $A_s$ and $A_f$, wherein $A_s$ is a temperature wherein austenitic transformation, namely transformation between the martensitic to the austenitic state, begins, and $A_f$ is a temperature where the austenitic transformation ends;

   (b) subjecting the raw NiTi alloy to a first heat, treatment based on results obtained in (a) so as to yield alloys with an initial internal structure condition having essentially equal random dislocation density wherein the first heat treatment is defined such that

   - where the difference is less than 7°C, heat treating the alloy to a temperature of 450-500° for 0.5-1.0 hours;

   - where the difference is more than 7°C, heat treating the alloy to a temperature of 510-550° for 1.0-2.5 hours,

   (c) subjecting the alloy to thermo-mechanical treatment (TMT) at a temperature above 0.3Tm where Tm is a melting temperature of the alloy in Kelvin, comprising plastic deformation of the alloy with simultaneous heating during a dynamic aging process, to yield a polygonal sub-grain dislocation structure decorated by precipitation;

   (d) if the deformation in step (c) did not yield the final form, subjecting the alloy to an intermediate heat treatment to conclude one cycle of sub-grain dislocation structure formation;

   (e) repeating steps (c) and (d) until yielding said final form; and

   (f) subjecting the alloy to a final heat and memorizing treatment, to yield two memory states consisting of an austenitic state, in which said alloy has an austenitic form and a martensitic state, the treatment comprising:

      (i) forming the alloy into said austenitic form; subjecting the alloy to a polygonization treatment in the range from 450 to 550°C to yield arrangement of random dislocation, then to solution treatment to release non-arranged dislocation from precipitation and provide for their rearrangement and then to an aging treatment; and deforming the alloy to assume a conditioning form with a higher degree of deformation than said martensitic form and heating to obtain the two memory states; or

      (ii) forming the alloy into a conditioning form with a higher degree of deformation than the martensitic form; subjecting said alloy to heat treatment, then to polygonization and solution treatment in the range from 600 to 800°C and then optionally to an aging treatment; forming the alloy into said austenitic form and subjecting the alloy to a memorizing heat treatment and to an aging treatment.

2.  A process according to claim 1, comprising the steps of:

(a) heating a sample of the raw NiTi alloy, to a temperature of 450-550°C for 0.5-2.5 hours and then testing the sample for temperature difference between $A_s$ and $A_f$,

(b) subjecting the raw NiTi alloy to a first heat treatment based on the $A_f$ - $A_s$ difference obtained in step (a), as follows:

-   where the difference is less than 7°C, heat treating the alloy to a temperature of 450-500° for 0.5-1.0 hours;

-   where the difference is more than 7°C, heat treating the alloy to a temperature of 510-550° for 1.0-2.5 hours;

(c) subjecting the alloy to TMT, comprising plastic deforming the alloy at a strain rate of less than 5 sec$^{-1}$, with simultaneous internal heating of a portion of the alloy where the deformation occurs to a temperature of 250-550°C, the deformation of this step being less than 55%;

(d) if the deformation in step (c) did not yield the final form, subjecting the alloy to an intermediate heat treatment at a temperature of 500-550°C for 0.5-2 hours, and then repeating step (c); and

(e) subjecting the alloy to a final heat and memorizing treatment, to yield two memory states consisting of an austenitic state, in which said alloy has an austenitic form and a martensitic state, the treatment comprising:

(i) forming the alloy into said austenitic form; subjecting the alloy to polygonization treatment in the range from 450 to 550°C to yield arrangement of random dislocation, then to solution treatment to release non-arranged dislocation from precipitation and provide for their arrangement and then to an aging treatment; and deforming the alloy to assume a conditioning form with a higher degree of deformation than said martensitic form and heating to obtain the two memory states; or

(ii) forming the alloy into conditioning form with a higher degree to deformation than the martensitic form; subjecting said alloy to heat treatment, then to polygonization and solution treatment in the range from 600 to 800°C and then optionally to an aging treatment; forming the alloy into said austenitic form; and subjecting the alloy to a memorizing heat treatment and to an aging treatment.

3.  A process according to claim 2, wherein the deformation step (c) is less than 40%.

4.  A process according to any of claim 1 to 3, wherein the final heat and memorizing treatment. comprises; (

(i) forming the alloy into said austenitic form;

(ii) subjecting the alloy to polygonization heat treatment at 450-550°C for 0.5-1.5 hours, then to a solution treatment at 600-800°C for 2-50 mins, and then to an aging treatment at 350-500° for 0.15-2.5 hours, and

(iii) deforming the alloy to assure a conditioning form, the deformation being less than 15% and being performed at a temperature T, which meets the following formula

$$T < M_s + 30°C$$

wherein $M_s$ is a temperature where the martensitic transformation begins, and then heating the alloy to a temperature of or above that in which the austenitic transformation of the alloy ends.

5.  A process according to claim 4, wherein the deformation of the alloy to assume the conditioning form in step (iii), is less than 7%.

6.  A process according to any one of claims 1 to 3, wherein the final heat and memorizing treatment comprises:

(i) forming the alloy into said conditioning form;

(ii) subjecting the alloy to a heat treatment at 450-500°C for 0.5-2 hours, then subjecting the alloy to polygonization and solution treatment at 600-800°C for 2-50 mins, and then subjecting the alloy to aging treatment at 350-500°C for 0-2 hours;

(iii) forming the alloy into the austenitic form; and

(iv) subjecting the alloy to a memorizing heat treatment at 500-600°C for more than 10 mins., and then subjecting the alloy to aging treatment at 350-500°C for 0.15-2.5 hours.

7. A process according to claim 4 to 6, comprising the following additional step:

(g) adjusting the temperature in which the austenitic transformation occurs, by either

- an aging treatment at a temperature of 350-500°C, to increase the temperature in which the austenitic transformation occurs, or

- a solution treatment at a temperature of 510-800°C to decrease the temperature in which the austenitic transformation occurs.

8. A process according to any of claims 1 to 7, wherein the thermo-mechanical treatment (TMT) comprises electro-stimulation with a current density of 500-2000 A/cm$^2$.

9. A process for preparing a medical device comprising a shape memory alloy (SMA) embodying a two-way shape memory effect, comprising treating a SMA in accordance with the process as defined in any one of claims 1 to 8.

10. A process according to claim 9, wherein said medical device is a stent.

11. A process for the manufacture of a medical stent from a NiTi alloy, being a wire having a first diameter, the stent having either the form of a wire with a second diameter or a form of a band, the stent exhibiting a two-way shape memory effect (SME) having an austenitic and a martensitic memory state with associated austenitic and martensitic shapes, respectively, the process comprising the steps of:

(a) heating a sample of the NiTi wire to a temperature of 450-550°C for 0.5-2.5 hours, and then testing the sample for temperature difference between $A_s$ and $A_f$, wherein $A_s$ is a temperature wherein austenitic transformation, namely transformation between the martensitic to the austenitic state, begins, and $A_f$ is a temperature where the austenitic transformation ends;

(b) subjecting the wire to a first heat treatment based on the $A_f$-$A_s$ difference obtained in step (a), as follows:

- where the difference is less than 7°C heat treating the wire to the temperature of 450-500°C for 0.5-1.0 hours:

- where the difference is more than about 7°C, heat treating the wire to a temperature of 510-550°C for 1.0-2.5 hours;

(c) subjecting the wire to a thermo-mechanical treatment, comprising warm rolling of the wire at a strain rate of less that 5 sec $^{-1}$, with simultaneous internal heating by electro-stimulation at a current density of 500-2000 A/cm$^2$, a portion of the wire where the deformation occurs, the deformation in this step being less than 55%;

(d) where the deformation in step (c) did not yield a cross-sectional shape of the final form, subjecting wire to an intermediate heat treatment at a temperature of 500-550°C, for 0.5-2 hours and then repeating step (c); and

(e) subjecting the wire to a final heat treatment and to a memorizing treatment which comprises:

(i) winding the wire or band obtained in step (c) on a mandrel having a diameter to be assured by the stent in the austenitic state,

(ii) subjecting the wire to a polygonization heat treatment at 450-550°C for 0.5-1.5 hours, then to a solution treatment at about 600-800°C for 2-50 mins, and then to an aging treatment at 350-500°C for 0.15-2.5 hours,

(iii) deforming the wire by winding it on a mandrel having a conditioning diameter, the deformation being less than about 7%, and being performed at a temperature T, which meets the following formula

$$T < M_s + 30°C$$

wherein $M_s$ is a temperature where the martensitic transformation begins, and then heating the wire or band to a temperature at or above that in which the austenitic transformation ends;

whereby a stent is obtained with an. austenitic state in which it has a diameter assumed in (i) above and a martensitic state in which it has a diameter which is an intermediate diameter between the conditioning diameter and the austenitic diameter.

12. A process for the manufacture of a medical stent from an NiTi wire having a first diameter, the stent having either the form of a wire with a second diameter or a form of a band, the stent exhibiting a two-way shape memory effect (SME) having an austenitic and a martensitic memory state with associated austenitic and martensitic shapes, respectively, the process comprising the steps of:

(a) heating a sample of a nickel-titanium based alloy wire to a temperature of 450-550°C for 0.5-2.5 hours, and then testing the sample for temperature difference between $A_s$ and $A_f$, wherein $A_s$ is a temperature wherein austenitic transformation, namely transformation between the martensitic to the austenitic state, begins, and $A_f$ is a temperature where the austenitic transformation ends;

(b) subjecting the wire to a first heat treatment based on the $A_s$ - $A_f$ difference obtained in step (a), as follows:

-   where the difference is less than about 7°C, heat treating the wire to a temperature of 450-500°C for 0.5-1.0 hours;

-   where the difference is more than 7°C, heat treating the wire to a temperature of 510-550°C for 1.0-2.5 hours;

(c) subjecting the wire to a thermo-mechanical treatment, comprising warm rolling of the wire at a strain rate of less than 5 sec $^{-1}$, with simultaneous internal heating of a portion of the wire where the deformation occurs, by electro-stimulation at a current density of 500-2000 A/cm$^2$, the deformation at this step being less than 55%;

(d) where the deformation is step (c) did not yield a cross-sectional shape of the final from, subjecting wire to an intermediate heat treatment at a temperature about 500-550°C for 0.2 hours and then repeating step (c); and

(e) subjecting the wire to a final heat treatment and to a memorizing treatment, which comprises:

(i) winding the wire or band obtained in step (c) on a mandrel having a conditioning diameter being different than the diameter to be assumed by the stent in the austenitic state,

(ii) subjecting the wire to a heat treatment at 450-500°C for 0.5-2 hours, then to a polygonization and solution treatment at 600-800°C for 2-50 mins, and then to aging treatment at 350-500°C for 0-2 hours,

(iii) winding the wire or band on a mandrel having a diameter to by assumed by the stent in the austenitic state,

(iv) subjecting the wire to a memorizing heat treatment at about 500-600°C for more than 10 mins., and then to aging treatment at 350-500°C for 0.15-2.15 hours;

whereby a stent is obtained having an austenitic state with a diameter into which the wire was formed in step (iii), and a martensitic state in which the stent has a diameter which is an intermediate diameter between the conditioning diameter and the diameter of the stent in the austenitic state.

13. A process for the manufacture of tooth root implant for NiTi alloy, exhibiting a two way shape memory effect having an austenitic and a martensitic memory state with associated austenitic and martensitic shapes, respectively, the process comprising the steps of:

(a) heating a sample of a NiTi rod to a temperature of 450-550°C for 0.5-2.5 hours, and then testing the sample for temperature difference between $A_s$ and $A_f$, wherein $A_s$ is a temperature wherein austenitic transformation, namely transformation between the martensitic to the austenitic state begins and $A_f$ is a temperature where the austenitic transformation ends;

(b) subjecting the rod to a first heat treatment based on the $A_f$-$A_s$ difference obtained in step (a), as follows:

-   where the difference is less than 7°C, heat treating the rod to a temperature of 450-500°C for 0.5-1.0 hours;

-   where the difference is more than 7°C, heat treating the rod to a temperature of 510-550°C for 1.0-2.5 hours;

(c) subjecting the rod to a thermo-mechanical treatment comprising warm drawing with a strain rate less than 5 sec$^{-1}$ with simultaneous heating, the total strain in this step being less than 55%;

(d) where the deformation in step (c) did not yield a cross-sectional shape of the final form, subjecting the rod to intermediate heat treatment at a temperature of 500-550°C for 0.5-2 hours and then repeating step (c);

(e) machining the rod to yield the shape of the implant;

(f) subjecting the implant to the final heat treatment and to a memorizing treatment which comprises:

(i) expanding implant's force segments to a diameter to be assumed by the implant in the austenitic state,

(ii) subjecting the implant to a polygonization heat treatment at 450-550°C for 0.5-1.5 hours, then to a solution treatment at 600-800°C for 2-50 min, and then to an aging treatment at about 350-500°C for 0.15-2.5 hours;

(iii) deforming the implant force segments to a conditioning diameter with a strain less than 7% and being performed at a temperature T < $M_s$ + 30°C, wherein $M_s$ is a temperature where the martensitic transformation begins and then heating the implant to a temperature at or above that in which the austenitic transformation ends;

whereby an implant is obtained with an austenitic state in which it has a diameter assumed in (i) above and a martensitic state in which it has a diameter, which is an intermediate diameter between the conditioning diameter and the austenitic diameter.

**14.** A process for the manufacture of a tube coupling from a NiTi alloy, exhibiting a two way shape memory effect having an austenitic and a martensitic memory states with associated austenitic and martensitic shapes, respectively, the process comprising the steps of:

(a) heating a sample of NiTi rod to a temperature of 450-550°C for 0.5-2.5 hours, and then testing the sample for temperature difference between $A_s$ and $A_f$, wherein $A_s$ is a temperature wherein austenitic transformation, namely transformation between the martensitic to the austenitic state, begins, and $A_f$ is a temperature where the austenitic transformation ends;

(b) subjecting the rod to a first heat treatment based on the $A_f$-$A_s$ difference obtained in step (a), as follows:

- where the difference is less than about 7°C, heat treating the rod to a temperature of 450-500°C for 0.5-2.0 hours;

- where the difference is more than 7°C, heat treating the wire to a temperature of 510-550°C for 1.0-2.5 hours;

(c) subjecting the rod to thermo-mechanical treatment comprising warm drawing with a strain rate of less than 5sec$^{-1}$ with simultaneous heating, the total strain being less than 55%;

(d) where the deformation in step (c) did not yield a cross-sectional shape of the final form, subjecting the rod to an intermediate heat treatment at a temperature of 500-550°C, for 0.5-2 hours and then repeating step (c); and

(e) machining the rod to yield coupling a hollow cylinder with a conditioning internal diameter;

(f) subjecting the cylinder to the final heat treatment and to a memorizing treatment, which comprises:

(i) subjecting the cylinder to a polygonization heat treatment at 450-550°C for about 0.5-1.5 hours, then to solution treatment at 600-800°C for 2-50 min. and then to an aging treatment at 350-500°C for 0-2.5 hours,

(ii) expanding the cylinder to a diameter to be assumed by the coupling in the austenitic state;

(iii) subjecting the cylinder to a memorizing heat treatment at 500-600°C for more than 10 mins., and then to an aging treatment at 350-500°C for 0.15-2.5 hours:

whereby a coupling is obtained having an austenitic state with a diameter into which it was formed in step (ii) and a martensitic state in which the coupling has a diameter which is an intermediate diameter between the conditioning internal diameter and the diameter of the coupling to the austenitic state.

**Patentansprüche**

**1.** Verfahren zur Bearbeitung einer NiTi-Legierung im unbehandelten Zustand mit einer Ausgangsform zur Bildung einer Legierung mit einer abschließenden Form, in welcher sie ein Formgedächtnis bzw. einen Shape-Memory-Effekt (SME) in zwei Richtungen aufweist, wodurch sie einen austenitischen und einen martensitischen Memory-Zustand mit zugehöriger austenitischer bzw. martensitischer Form aufweist, wobei das Verfahren in Kombination die folgenden Schritte aufweist:

(a) Prüfen der Legierung auf NiTi-Basis im unbehandelten Zustand, um so durch Messen des Unterschiedes zwischen $A_s$ und $A_f$ einen abgeschätzten Aufschluss über die innere Struktur der Legierung zu erhalten, wobei $A_s$ eine Temperatur ist, bei welcher die austenitische Transformation, nämlich die Transformation aus dem martensitischen Zustand in den austenitischen Zustand, einsetzt, und $A_f$ eine Temperatur ist, bei welcher die austenitische Transformation beendet ist;

(b) Vornehmen einer ersten Wärmebehandlung bei der NiTi-Legierung im unbehandelten Zustand, welcher die im Schritt (a) erhaltenen Ergebnisse zugrunde liegen, um so Legierungen mit einer anfänglichen inneren Struktur zu erhalten, welche eine im wesentlichen gleiche zufällige Versetzungsdichte aufweisen, wobei die erste Wärmebehandlung in der Weise definiert ist, dass

- in den Fällen, in denen der Unterschied weniger als 7 °C beträgt, die Wärmebehandlung der Legierung auf eine Temperatur von 450 - 500 ° über 0,5 bis 1,0 Stunden erfolgt;

- in den Fällen, in denen der Unterschied mehr als 7 °C beträgt, die Wärmebehandlung der Legierung auf eine Temperatur von 510 - 550 ° über 1,0 bis 2,5 Stunden erfolgt;

(c) Vornehmen einer thermomechanischen Behandlung (TMT) bei der Legierung bei einer Temperatur von über 0,3 Tm, wobei Tm die Schmelztemperatur der Legierung in Kelvin ist, unter Einbeziehung der plastischen Verformung der Legierung bei gleichzeitiger Erwärmung während eines dynamischen Alterungsprozesses, um eine polygone Verschiebungsstruktur im Subkombereich zu erzielen, die durch Ausfällung dekoriert ist;

(d) falls die Verformung im Schritt (c) nicht die abschließende Form erbracht hat, Vornehmen einer zwischengeschalteten Wärmebehandlung bei der Legierung einer zum Abschließen eines Zyklus zur Bildung der Verschiebungsstruktur im Subkornbereich;

(e) Wiederholen der Schritte (c) und (d), bis die abschließende Form erhalten wurde; und

(f) Vornehmen einer abschließenden Wärmebehandlung bei der Legierung mit Ausbildung des Memory-Effekts, um zwei Memory-Zustände zu erhalten, die aus einem austenitischen Zustand, in dem die Legierung eine austenitische Form aufweist, und einem martensitischen Zustand besteht, wobei diese Behandlung folgende Schritte umfasst:

(I) Umformen der Legierung zu der austenitischen Form; Vornahme einer Behandlung zur Polygonisation bei der Legierung im Bereich von 450 bis 550 °C, um eine Anordnung mit zufälliger Verschiebung zu erzielen, dann Behandeln der Legierung mit einer Lösung zur Freisetzung einer ungeordneten Verschiebung aus der Ausfällung und um für deren Neuordnung zu sorgen, sowie anschließend Bearbeitung zur Alterung; und Verformen der Legierung, damit diese eine Konditionierungsform mit einem höheren Grad der Verformung als bei der martensitischen Form annimmt, und mit Erwärmung, um die beiden Memory-Zustände herbeizuführen; oder

(II) Umformen der Legierung zu einer Konditionierungsform mit einem höheren Grad der Verformung als bei der martensitischen Form; Vornahme einer Wärmebehandlung mit anschließender Polygonisation bei der Legierung und Behandeln der Legierung mit einer Lösung im Bereich von 600 bis 800 °C, und anschließend bei Bedarf Vornehmen einer Bearbeitung zur Alterung bei der Legierung; Umformen der Legierung zu der austenitischen Form und Vornehmen einer Wärmebehandlung bei der Legierung zur Erzielung des Memory-Effekts, sowie einer Behandlung zur Alterung.

2. Verfahren nach Anspruch 1, welches die folgenden Schritte umfasst:

(a) Erwärmen einer Probe der NiTi-Legierung im unbehandelten Zustand auf eine Temperatur von 450 bis 550 °C über 0,5 bis 2,5 Stunden lang, und anschließendes Untersuchen der Probe auf eine Temperaturdifferenz zwischen $A_s$ und $A_f$,

(b) Vornehmen einer ersten Wärmebehandlung der NiTi-Legierung im unbehandelten Zustand auf der Grundlage der Differenz $A_f - A_s$, die im Schritt (a) ermittelt wurde, auf die folgende Weise:

- in den Fällen, in denen der Unterschied weniger als 7 °C beträgt, erfolgt die Wärmebehandlung der Legierung auf eine Temperatur von 450 - 500 ° über 0,5 bis 1,0 Stunden;

- in den Fällen, in denen der Unterschied mehr als 7 °C beträgt, erfolgt die Wärmebehandlung der Legierung auf eine Temperatur von 510 - 550 ° über 1,0 bis 2,5 Stunden;

(c) Vornehmen einer TMT-Behandlung bei der Legierung unter Einbeziehung einer plastischen Verformung der Legierung mit einer Verformungsgeschwindigkeit von weniger als 5 sec$^{-1}$ bei gleichzeitiger Erwärmung im Inneren eines Teils der Legierung, in welchem die Verformung bis zu einer Temperatur von 250 bis 550 °C auftritt, wobei die Verformung bei diesem Schritt weniger als 55 % beträgt;

(d) falls die Verformung im Schritt (c) nicht die abschließende Form erbracht hat, Vornehmen einer zwischengeschalteten Wärmebehandlung bei der Legierung bei einer Temperatur von 500 bis 550 °C über 0,5 bis 2 Stunden und anschließendes Wiederholen des Schritts (c); und

(e) Vornehmen einer abschließenden Wärmebehandlung bei der Legierung mit Ausbildung des Memory-Effekts, um zwei Memory-Zustände zu erhalten, die aus einem austenitischen Zustand, in dem die Legierung eine austenitische Form aufweist, und einem martensitischen Zustand besteht, wobei diese Behandlung folgende Schritte umfasst:

(I) Umformen der Legierung zu der austenitischen Form; Vornehmen einer Behandlung der Legierung zur Polygonisation im Bereich von 450 bis 550 °C, um eine Anordnung mit zufälliger Verschiebung zu erzielen, dann Behandeln der Legierung mit einer Lösung zur Freisetzung einer ungeordneten Verschiebung aus der Ausfällung und um für deren Neuordnung zu sorgen, sowie anschließend Behandlung der Legierung zur Alterung; und Verformen der Legierung, damit diese eine Konditionierungsform mit einem höheren Grad der Verformung als bei der martensitischen Form annimmt, und mit Erwärmung, um die beiden Memory-Zustände herbeizuführen; oder

(II) Umformen der Legierung zu einer Konditionierungsform mit einem höheren Grad der Verformung als bei der martensitischen Form; Vornahme einer Wärmebehandlung bei der Legierung mit anschließender Polygonisation und Behandeln der Legierung mit einer Lösung im Bereich von 600 bis 800 °C, und anschließend bei Bedarf Vornahme einer Behandlung zur Alterung bei der Legierung; Umformen der Legierung zu der austenitischen Form und Vornehmen einer Wärmebehandlung bei der Legierung zur Erzielung des Memory-Effekts, sowie einer Behandlung zur Alterung.

3. Verfahren nach Anspruch 2, bei welchem im Schritt (c) zur Umformung die Verformung weniger als 40 % beträgt.

4. Verfahren nach einem der Ansprüche 1 bis 3, bei welchem die abschließende Wärmebehandlung mit Behandlung zur Herbeiführung des Memory-Effekts die folgenden Schritte umfasst:

(I) Umformen der Legierung zu der austenitischen Form;

(II) Vornehmen einer Wärmebehandlung der Legierung zur Polygonisation über 0,5 bis 1,5 Stunden lang bei 450 bis 550 °C, anschließend Behandeln der Legierung mit einer Lösung über 2 bis 50 Minuten lang bei 600 bis 800 °C, und anschließendes Vornehmen einer Behandlung zur Alterung über 0,15 bis 2,5 Stunden lang bei 350 bis 500 °C, und

(III) Umformen der Legierung zur Sicherstellung einer Konditionierungsform, wobei die Verformung weniger als 15 % beträgt und bei einer Temperatur T vorgenommen wird, welche der folgenden Formel entspricht:

$$T < M_s + 30°C$$

wobei $M_s$ eine Temperatur darstellt, bei welcher die martensitische Transformation einsetzt, und anschließendes Erwärmen der Legierung auf eine Temperatur, die der Temperatur entspricht oder über dieser liegt, bei der die austenitische Transformation der Legierung beendet ist.

5. Verfahren nach Anspruch 4, bei welchem die Verformung der Legierung zur Umformung in die Konditionierungsform im Schritt (III) weniger als 7 % beträgt.

6. Verfahren nach einem der Ansprüche 1 bis 3, bei welchem die abschließende Wärmebehandlung mit Herbeiführung des Memory-Effekts folgendes umfasst:

(I) Umformen der Legierung zu der Konditionierungsform;

(II) Vornehmen einer Wärmebehandlung der Legierung über 0,5 bis 1,5 Stunden lang bei 450 bis 550 °C, anschließend Behandeln der Legierung zur Polygonisation und Behandeln derselben mit einer Lösung über 2 bis 50 Minuten lang bei 600 bis 800 °C, und anschließendes Vornehmen einer Behandlung zur Alterung über 0 bis 2 Stunden lang bei 350 bis 500 °C;

(III) Umformen der Legierung zur austenitischen Form; und

(IV) Vornehmen einer Wärmebehandlung der Legierung mit Herbeiführen des Memory-Effekts über eine längere Zeit als 10 Minuten bei 500 bis 600 °C, und anschließendes Vornehmen einer Behandlung zur Alterung über 0,15 bis 2,5 Stunden lang bei 350 bis 500 °C.

7. Verfahren nach den Ansprüchen 4 bis 6, welches den folgenden zusätzlichen Schritt umfasst:

(g) Einstellen der Temperatur, bei welcher die austenitische Transformation eintritt, durch eine der beiden folgenden Maßnahmen:

- eine Behandlung zur Alterung bei einer Temperatur von 350 bis 500 °C, um die Temperatur zu erhöhen, bei welcher die austenitische Transformation eintritt, oder
- Behandlung mit einer Lösung bei einer Temperatur von 510 bis 800 °C, um die Temperatur zu senken, bei welcher die austenitische Transformation auftritt.

8. Verfahren nach einem der Ansprüche 1 bis 7, bei welchem die thermomechanische Behandlung (TMT) die Elektrostimulierung mit einer Stromdichte von 500 bis 2000 A/cm$^2$ umfasst.

9. Verfahren zur Herstellung einer Vorrichtung für den Einsatz in der Medizin, welches eine Legierung mit Formgedächtnis (SMA) aufweist, die einen Shape-Memory-Effekt in zwei Richtungen verkörpert, wobei das Verfahren die Behandlung einer SMA-Legierung gemäß dem Verfahren nach einem der Ansprüche 1 bis 8 umfasst.

10. Verfahren nach Anspruch 9, bei welchem es sich bei der Vorrichtung für den Einsatz in der Medizin ein Stent ist.

11. Verfahren zur Herstellung eines Stents zum Einsatz in der Medizin aus einer NiTi-Legierung, die dabei in Form eines Drahts mit einem ersten Durchmesser vorliegt, wobei der Stent entweder die Form eines Drahts mit einem zweiten Durchmesser oder die Form eines Streifens aufweist und wobei der Stent einen Shape-Memory-Effekt in zwei Richtungen (SME) mit einem austenitischen Memory-Zustand und einem martensitischen Memory-Zustand mit zugehöriger austenitischer Form bzw. martensitischer Form aufweist, wobei das Verfahren die folgenden Schritte umfasst:

(a) Erwärmen einer Probe des NiTi-Drahtes über 0,5 bis 2,5 Stunden lang auf eine Temperatur von 450 bis 550 °C, und anschließendes Untersuchen der Probe auf eine Temperaturdifferenz zwischen $A_s$ und $A_f$, wobei $A_s$ eine Temperatur ist, bei welcher die austenitische Transformation, nämlich die Transformation aus dem martensitischen Zustand in den austenitischen Zustand einsetzt, und $A_f$ eine Temperatur ist, bei welcher die austenitische Transformation beendet ist;

(b) Vornehmen einer ersten Wärmebehandlung des Drahtes auf der Grundlage der Differenz $A_f$ - $A_s$, die im Schritt (a) ermittelt wurde, auf die folgende Weise:

- in den Fällen, in denen der Unterschied weniger als 7 °C beträgt, erfolgt die Wärmebehandlung des Drahtes auf eine Temperatur von 450 - 500 °C über 0,5 bis 1,0 Stunden;
- in den Fällen, in denen der Unterschied mehr als 7 °C beträgt, erfolgt die Wärmebehandlung des Drahtes auf eine Temperatur von 510 - 550 °C über 1,0 bis 2,5 Stunden;

(c) Vornehmen einer thermomechanischen Behandlung an dem Draht unter Einbeziehung eines Arbeitsgangs zum Warmwalzen des Drahtes bei einer Verformungsgeschwindigkeit von weniger als 5 sec$^{-1}$ bei gleichzeitiger Erwärmung durch Elektrostimulation mit einer Stromdichte von 500 bis 2000 A/cm$^2$ im Inneren eines Teils des Drahtes, in welchem die Verformung auftritt, wobei die Verformung bei diesem Schritt weniger als 55 % beträgt;

(d) falls die Verformung im Schritt (c) nicht eine Querschnittsform der abschließenden Form erbracht hat, Vornehmen einer zwischengeschalteten Wärmebehandlung bei dem Draht bei einer Temperatur von 500 bis 550 °C über 0,5 bis 2 Stunden und anschließendes Wiederholen des Schritts (c); und

(e) Vornehmen einer abschließenden Wärmebehandlung bei dem Draht mit Ausbildung des Memory-Effekts, wobei diese Behandlung folgende Schritte umfasst:

(I) Aufwickeln des in Schritt (c) erhaltenen Drahtes bzw. Streifens auf einen Dorn mit einem Durchmesser, der im austenitischen Zustand von dem Stent zu gewährleisten ist,

(II) Vornehmen einer Wärmebehandlung des Drahtes zur Polygonisation bei 450 bis 550 °C über 0,5 bis 1,5 Stunden lang, dann Behandeln des Drahtes mit einer Lösung bei etwa 600 bis 800 °C über 2 bis 50 Minuten lang, sowie anschließend Behandlung des Drahtes zur Alterung bei 350 bis 500 °C über 0,15 bis 2,5 Stunden lang,

(III) Verformen des Drahtes durch Aufwickeln desselben auf einen Dom mit einem Konditionierungsdurchmesser, wobei die Verformung weniger als etwa 7 % beträgt und bei einer Temperatur T vorgenommen wird, welche der folgenden Formel entspricht:

$$T < M_s + 30°C$$

wobei $M_s$ eine Temperatur darstellt, bei welcher die martensitische Transformation einsetzt, und anschließendes Erwärmen des Drahtes bzw. Streifens auf eine Temperatur, die der Temperatur entspricht oder über dieser liegt, bei der die austenitische Transformation beendet ist.

wodurch man einen Stent mit einem austenitischen Zustand erhält, bei dem er einen im vorstehenden Punkt (I) angenommenen Durchmesser aufweist, und mit einem martensitischen Zustand, in dem er einen Durchmesser aufweist, der einem Wert zwischen dem Konditionierungsdurchmesser und dem Durchmesser im austenitischen Zustand entspricht.

12. Verfahren zur Herstellung eines Stents zum Einsatz in der Medizin aus einem NiTi-Draht mit einem ersten Durchmesser, wobei der Stent entweder die Form eines Drahtes mit einem zweiten Durchmesser oder die Form eines Streifens aufweist und wobei der Stent ein Formgedächtnis (SME) in zwei Richtungen mit einem austenitischen und einem martensitischen Gedächtniszustand mit der zugehörigen jeweiligen austenitischen bzw. martensitischen Form besitzt, wobei das Verfahren die folgenden Schritte umfasst:

(a) Erwärmen einer Probe eines Drahtes aus einer Legierung auf der Basis von Nickel-Titan über 0,5 bis 2,5 Stunden lang auf eine Temperatur von 450 bis 550 °C, und anschließendes Untersuchen der Probe auf eine Temperaturdifferenz zwischen $A_s$ und $A_f$, wobei $A_s$ eine Temperatur ist, bei welcher die austenitische Transformation, nämlich die Transformation aus dem martensitischen Zustand in den austenitischen Zustand einsetzt, und $A_f$ eine Temperatur ist, bei welcher die austenitische Transformation beendet ist;

(b) Vornehmen einer ersten Wärmebehandlung des Drahtes auf der Grundlage der Differenz $A_f$ - $A_s$, die im Schritt (a) ermittelt wurde, auf die folgende Weise:

- in den Fällen, in denen der Unterschied weniger als 7 °C beträgt, erfolgt die Wärmebehandlung des Drahtes auf eine Temperatur von 450 - 500 ° über 0,5 bis 1,0 Stunden;
- in den Fällen, in denen der Unterschied mehr als 7 °C beträgt, erfolgt die Wärmebehandlung des Drahtes auf eine Temperatur von 510 - 550 ° über 1,0 bis 2,5 Stunden;

(c) Vornehmen einer thermomechanischen Behandlung an dem Draht unter Einbeziehung eines Arbeitsgangs zum Warmwalzen des Drahtes bei einer Verformungsgeschwindigkeit von weniger als 5 sec$^{-1}$ bei gleichzeitiger Erwärmung durch Elektrostimulation mit einer Stromdichte von 500 bis 2000 A/cm$^2$ im Inneren eines Teils des Drahtes, in welchem die Verformung auftritt, wobei die Verformung bei diesem Schritt weniger als 55 % beträgt;

(d) falls die Verformung im Schritt (c) nicht eine Querschnittsform der abschließenden Form erbracht hat, Vornehmen einer zwischengeschalteten Wärmebehandlung bei dem Draht 0,2 Stunden lang bei einer Temperatur von 500 bis 550 °C und anschließendes Wiederholen des Schritts (c); und

(e) Vornehmen einer abschließenden Wärmebehandlung bei dem Draht mit Ausbildung des Memory-Effekts, wobei diese Behandlung folgende Schritte umfasst:

(I) Aufwickeln des in Schritt (c) erhaltenen Drahtes bzw. Streifens auf einen Dom mit einem Konditionierungsdurchmesser, der sich von dem Durchmesser unterscheidet, den der Stent im austenitischen Zustand einnehmen soll,

(II) Vornehmen einer Wärmebehandlung des Drahtes bei 450 bis 550 °C über 0,5 bis 2 Stunden lang, anschließend Behandlung zur Polygonisation und dann Behandeln des Drahtes mit einer Lösung bei etwa 600 bis 800 °C über 2 bis 50 Minuten lang, sowie anschließend Behandlung des Drahtes zur Alterung bei 350 bis 500 °C über 0 bis 2 Stunden lang,

(III) Aufwickeln des Drahtes bzw. Streifens auf einen Dom mit einem Durchmesser, den der Stent im austenitischen Zustand einnehmen soll,

(IV) Wärmebehandlung des Drahtes mit Ausbildung des Memory-Effekts bei etwa 500 bis 600 °C über einen Zeitraum von mehr als 10 Minuten, sowie anschließend Behandlung des Drahtes zur Alterung bei 350 bis 500 °C über 0,15 bis 2,5 Stunden lang;

wodurch man einen Stent mit einem austenitischen Zustand erhält, in dem er einen Durchmesser besitzt, zu dem der Draht im Schritt (III) geformt wurde, sowie mit einem martensitischen Zustand, in dem er einen Durchmesser aufweist, der einem Wert zwischen dem Konditionierungsdurchmesser und dem Durchmesser des Stents im austenitischen Zustand entspricht.

13. Verfahren zur Herstellung eines Zahnwurzel-Implantats aus einer NiTi-Legierung, welche ein Formgedächtnis (SME) in zwei Richtungen mit einem austenitischen und einem martensitischen Gedächtniszustand mit der zugehörigen jeweiligen austenitischen bzw. martensitischen Form besitzt, wobei das Verfahren die folgenden Schritte umfasst:

(a) Erwärmen einer Probe eines Stabs aus einer Ni-Ti-Legierung über 0,5 bis 2,5 Stunden lang auf eine Temperatur von 450 bis 550 °C, und anschließendes Untersuchen der Probe auf eine Temperaturdifferenz zwischen $A_s$ und $A_f$, wobei $A_s$ eine Temperatur ist, bei welcher die austenitische Transformation, nämlich die Transformation aus dem martensitischen Zustand in den austenitischen Zustand einsetzt, und $A_f$ eine Temperatur ist, bei welcher die austenitische Transformation beendet ist;

(b) Vornehmen einer ersten Wärmebehandlung des Stabes auf der Grundlage der Differenz $A_f - A_s$, die im Schritt (a) ermittelt wurde, auf die folgende Weise:

- in den Fällen, in denen der Unterschied weniger als 7 °C beträgt, erfolgt die Wärmebehandlung des Drahtes auf eine Temperatur von 450 - 500 °C über 0,5 bis 1,0 Stunden;

- in den Fällen, in denen der Unterschied mehr als 7 °C beträgt, erfolgt die Wärmebehandlung des Drahtes auf eine Temperatur von 510 - 550 °C über 1,0 bis 2,5 Stunden;

(c) Vornehmen einer thermomechanischen Behandlung an dem Stab unter Einbeziehung eines Arbeitsgangs zum Warmziehen des Stabs bei einer Verformungsgeschwindigkeit von weniger als 5 sec$^{-1}$ bei gleichzeitiger Erwärmung, wobei die gesamte Zugumformung in diesem Schritt weniger als 55 % beträgt;

(d) falls die Verformung im Schritt (c) nicht eine Querschnittsform der abschließenden Form erbracht hat, Vornehmen einer zwischengeschalteten Wärmebehandlung bei dem Stab 0,5 bis 2 Stunden lang bei einer Temperatur von 500 bis 550 °C und anschließendes Wiederholen des Schritts (c); und

(e) maschinelle Bearbeitung des Stabs, um die Form des Implantats zu erhalten;

(f) Vornehmen einer abschließenden Wärmebehandlung an den Stab mit Ausbildung des Memory-Effekts, wobei diese Behandlung folgende Schritte umfasst:

(I) Aufweiten der Kraftsegmente des Implantats auf einen Durchmesser, den das Implantat im austenitischen Zustand einnehmen soll,

(II) Vornehmen einer Wärmebehandlung des Implantats zur Polygonisation bei 450 bis 550 °C über 0,5 bis 1,5 Stunden lang, anschließend Behandeln des Implantats mit einer Lösung bei etwa 600 bis 800 °C über 2 bis 50 Minuten lang, sowie danach Behandlung des Implantats zur Alterung bei 350 bis 500 °C über 0,15 bis 2,5 Stunden lang,

(III) Verformen der Kraftsegmente des Implantats auf einen Konditionierungsdurchmesser bei einer Zugumformung von weniger als 7 % , wobei der Vorgang bei einer Temperatur von $T < M_s + 30$ °C vorgenommen wird, wobei Ms eine Temperatur angibt, bei welcher die martensitische Transformation einsetzt, und wobei das Implantat dann auf eine Temperatur erwärmt wird, die bei dem Wert oder über diesem liegt, bei welchem die austenitische Transformation beendet ist;

wodurch man ein Implantat mit einem austenitischen Zustand erhält, in dem es einen Durchmesser besitzt, den es im Schritt (I) angenommen hat, sowie mit einem martensitischen Zustand, in dem es einen Durchmesser aufweist, der einem Wert zwischen dem Konditionierungsdurchmesser und dem Durchmesser des Stents im austenitischen Zustand entspricht.

14. Verfahren zur Herstellung einer Schlauchkupplung aus einer NiTi-Legierung, welche ein Formgedächtnis (SME) in zwei Richtungen mit einem austenitischen und einem martensitischen Gedächtniszustand mit der zugehörigen jeweiligen austenitischen bzw. martensitischen Form besitzt, wobei das Verfahren die folgenden Schritte umfasst:

(a) Erwärmen einer Probe eines NiTi-Stabs über 0,5 bis 2,5 Stunden lang auf eine Temperatur von 450 bis 550 °C, und anschließendes Untersuchen der Probe auf eine Temperaturdifferenz zwischen $A_s$ und $A_f$, wobei $A_s$ eine Temperatur ist, bei welcher die austenitische Transformation, nämlich die Transformation aus dem martensitischen Zustand in den austenitischen Zustand einsetzt, und $A_f$ eine Temperatur ist, bei welcher die austenitische Transformation beendet ist;

(b) Vornehmen einer ersten Wärmebehandlung des Stabs auf der Grundlage der Differenz $A_f$ - $A_s$, die im Schritt (a) ermittelt wurde, auf die folgende Weise:

- in den Fällen, in denen der Unterschied weniger als 7 °C beträgt, erfolgt die Wärmebehandlung des Drahtes auf eine Temperatur von 450 - 500 °C über 0,5 bis 1,0 Stunden;

- in den Fällen, in denen der Unterschied mehr als 7 °C beträgt, erfolgt die Wärmebehandlung des Drahtes auf eine Temperatur von 510 - 550 °C über 1,0 bis 2,5 Stunden;

(c) Vornehmen einer thermomechanischen Behandlung an dem Draht unter Einbeziehung eines Arbeitsgangs zum Warmziehen bei einer Verformungsgeschwindigkeit von weniger als 5 $sec^{-1}$ bei gleichzeitiger Erwärmung, wobei die gesamte Zugumformung weniger als 55 % beträgt;

(d) falls die Verformung im Schritt (c) nicht eine Querschnittsform der abschließenden Form erbracht hat, Vornehmen einer zwischengeschalteten Wärmebehandlung bei dem Stab 0,5 bis 2 Stunden lang bei einer Temperatur von 500 bis 550 °C und anschließendes Wiederholen des Schritts (c); und

(e) maschinelle Bearbeitung des Stabs, um eine Kupplung in Form eines Hohlzylinders mit einem Innendurchmesser zur Konditionierung zu erhalten;

(f) Vornehmen einer abschließenden Wärmebehandlung an dem Zylinder mit Ausbildung des Memory-Effekts, wobei diese Behandlung folgende Schritte umfasst:

(I) Vornehmen einer Wärmebehandlung des Zylinders zur Polygonisation bei 450 bis 550 °C über etwa 0,5 bis 1,5 Stunden lang, anschließend Behandeln des Zylinders mit einer Lösung bei etwa 600 bis 800 °C über 2 bis 50 Minuten lang, sowie anschließend Behandlung des Zylinders zur Alterung bei 350 bis 500 °C über 0 bis 2,5 Stunden lang,

(II) Aufweiten des Zylinders auf einen Durchmesser, den die Kupplung im austenitischen Zustand einnehmen soll;

(III) Wärmebehandlung des Zylinders mit Ausbildung des Memory-Effekts bei etwa 500 bis 600 °C über einen Zeitraum von mehr als 10 Minuten, sowie anschließend Behandlung des Drahtes zur Alterung bei 350 bis 500 °C über 0,15 bis 2,5 Stunden lang;

wodurch man eine Kupplung mit einem austenitischen Zustand erhält, in dem sie einen Durchmesser besitzt, auf den sie im Schritt (II) geformt wurde, sowie mit einem martensitischen Zustand, in dem sie einen Durchmesser aufweist, der einem Wert zwischen dem Konditionierungs-Innendurchmesser und dem Durchmesser der Kupplung im austenitischen Zustand entspricht.

## Revendications

1. Procédé pour traiter un alliage brut de NiTi ayant une forme initiale pour obtenir un alliage avec une forme finale dans lequel il possède un double effet de mémoire de forme (SME) moyennant quoi il présente un état de mémoire austénitique et martensitique avec des formes austénitiques et martensitiques associées, respectivement, le procédé combinant les étapes consistant à :

(a) tester l'alliage brut à base de NiTi afin d'obtenir une estimation de la structure interne de l'alliage en mesurant la différence entre $A_s$ et $A_f$, dans lequel $A_s$ est une température à laquelle la transformation austénitique, c'est-à-dire la transformation entre l'état martensitique et l'état austénitique, commence, et $A_f$ est une température à laquelle la transformation austénitique se termine ;

(b) soumettre l'alliage brut de NiTi à un premier traitement thermique basé sur les résultats obtenus à l'étape (a) afin de produire des alliages avec une condition de structure interne initiale ayant une densité de dislocation aléatoire sensiblement égale, dans laquelle le premier traitement thermique est défini de sorte que chaque fois que la différence est inférieure à 7°C, traiter thermiquement l'alliage à une température de 460 à 500°C pendant 0,5 à 1,0 heure ; chaque fois que la différence est supérieure à 7°C, traiter thermiquement l'alliage à une température de 510 à 550 °C pendant 1,0 à 2,5 heures ;

(c) soumettre l'alliage au traitement thermomécanique (TMT) à une température située au dessus de 0,3 $T_M$ , où $T_M$ représente une température de fusion de l'alliage exprimée en Kelvin, comprenant la déformation plastique de l'alliage en chauffant simultanément pendant un processus de vieillissement dynamique, pour produire une structure de dislocation à sous-grain polygonal décorée par précipitation ;

(d) si la déformation à l'étape (c) ne produit pas la forme finale, soumettre l'alliage à un traitement thermique intermédiaire pour conclure un cycle de formation de structure de dislocation à sous-grain ;

(e) répéter les étapes (c) et (d) jusqu'à la production de ladite forme finale ; et

(f) soumettre l'alliage à un traitement thermique et à mémoire final, pour produire deux états de mémoire qui se composent d'un état austénitique, dans lequel ledit alliage a une forme austénitique et d'un état martensitique, le traitement comprenant les étapes consistant à :

(i) former l'alliage dans ladite forme austénitique ; soumettre l'alliage à un traitement de polygonisation dans les limites d'une température comprise entre 450 et 550°C pour produire l'agencement de dislocation aléatoire, ensuite à un traitement de mise en solution pour libérer la dislocation non agencée de la précipitation et prévoir leur réagencement et ensuite à un traitement de vieillissement ; et déformer l'alliage pour prendre une forme de conditionnement avec un degré supérieur de déformation par rapport à ladite forme martensitique et chauffer pour obtenir les deux états de mémoire ; ou

(ii) former l'alliage dans une forme de conditionnement avec un degré supérieur de déformation par rapport à la forme martensitique ; soumettre ledit alliage au traitement thermique, ensuite à la polygonisation et au traitement de mise en solution dans les limites d'une température comprise entre 600 et 800°C, et ensuite facultativement à un traitement de vieillissement ; former l'alliage dans ladite forme austénitique et soumettre l'alliage à un traitement thermique à mémoire et à un traitement de vieillissement.

**2.** Procédé selon la revendication 1, comprenant les étapes consistant à :

(a) chauffer un échantillon d'alliage brut de NiTi à une température de 450 à 550°C pendant 0,5 à 2,5 heures et tester ensuite l'échantillon concernant la différence de température entre $A_s$ et $A_f$;

(b) soumettre l'alliage brut de NiTi à un premier traitement thermique basé sur la différence $A_f$ - $A_s$ obtenue à l'étape (a), de la manière suivante :

lorsque la différence est inférieure à 7°C, traiter thermiquement l'alliage à une température de 450 à 550°C pendant 0,5 à 1,0 heure ;
lorsque la différence est supérieure à 7°C, traiter thermiquement l'alliage à une température de 510 à 550°C pendant 1,0 à 2,6 heures ;

(c) soumettre l'alliage au TMT, comprenant la déformation plastique de l'alliage à une vitesse de déformation inférieure à 5 sec$^{-1}$, avec le chauffage interne simultané d'une partie de l'alliage où la déformation se produit à une température de 250 à 550°C, la déformation de cette étape étant inférieure à 55% ;

(d) si la déformation à l'étape (c) ne produit pas la forme finale, soumettre l'alliage à un traitement thermique intermédiaire à une température de 500 à 550°C pendant 0,5 à 2 heures, et répéter ensuite l'étape (c) ; et

(e) soumettre l'alliage à un traitement thermique et à mémoire final, pour produire deux états de mémoire qui se composent d'un état austénitique, dans lequel ledit alliage a une forme austénitique et d'un état martensitique, le traitement comprenant les étapes consistant à :

(i) former l'alliage dans ladite forme austénitique ; soumettre l'alliage au traitement de polygonisation dans les limites d'une température comprise entre 450 et 550°C pour produire l'agencement de la dislocation aléatoire, ensuite au traitement de mise en solution pour libérer la dislocation non agencée de le précipitation et prévoir leur agencement et ensuite à un traitement de vieillissement ; et déformer l'alliage pour prendre une forme de conditionnement avec un degré supérieur de déformation par rapport à ladite forme martensitique et chauffer pour obtenir les deux états de mémoire ; ou

(ii) former l'alliage dans la forme de conditionnement avec un degré supérieur de déformation par rapport à la forme martensitique ; soumettre ledit alliage au traitement thermique ; ensuite à la polygonisation et au traitement de mise en solution dans les limites d'une température comprise entre 600 et 800°C, et ensuite facultativement à un traitement de vieillissement ; former l'alliage dans ladite forme austénitique ; et soumettre l'alliage à un traitement thermique à mémoire et à un traitement de vieillissement.

3.  Procédé selon la revendication 2, dans lequel la déformation de l'étape (c) est inférieure à 40%.

4.  Procédé selon l'une quelconque des revendications 1 à 3, dans lequel le traitement thermique et à mémoire final comprend les étapes consistant à :

(i) former l'alliage dans ladite forme austénitique ;
(ii) soumettre l'alliage au traitement thermique de polygonisation à 450 à 550°C pendant 0,5 à 1,5 heures, ensuite à un traitement de mise en solution à 600 à 800°C pendant 2 à 50 minutes et ensuite à un traitement de vieillissement à 350 à 500°C pendant 0,15 à 2,5 heures ; et
(iii) déformer l'alliage pour prendre une forme de conditionnement, la déformation étant inférieure à 15% et étant réalisée à une température T, qui satisfait la formule suivante :

$$T < M_s + 30°C$$

dans laquelle $M_s$ est une température à laquelle la transformation martensitique commence, et ensuite chauffer l'alliage à une température de ou supérieure à celle à laquelle la transformation austénitique de l'alliage se termine.

5.  Procédé selon la revendication 4, dans lequel la déformation de l'alliage pour prendre la forme de conditionnement à l'étape (iii) est inférieure à 7%.

6.  Procédé selon l'une quelconque des revendications 1 à 3, dans lequel le traltement thermique et à mémoire final comprend les étapes consistant à :

(i) former l'alliage dans ladite forme de conditionnement ;
(ii) soumettre l'alliage à un traitement thermique à 450 à 500°C pendant 0,5 à 2 heures, soumettre ensuite l'alliage à la polygonisation et au traitement de mise en solution à 600 à 800°C pendant 2 à 50 minutes, et soumettre ensuite l'alliage au traitement de vieillissement à 350 à 500°C pendant 0 à 2 heures ;
(iii) former l'alliage dans la forme austénitique ; et
(iv) soumettre l'alliage à un traitement thermique à mémoire à 500 à 600°C pendant plus de 10 minutes, et soumettre ensuite l'alliage au traitement de vieillissement à 350 à 500°C pendant 0,15 à 2,5 heures.

7.  Procédé selon les revendications 4 à 6, comprenant l'étape supplémentaire suivante consistant à :

(g) régler la température à laquelle le transformation austénitique se produit, soit par :

un traitement de vleillissement à une température de 360 à 500°C pour augmenter la température à laquelle la transformation austénitique se produit, ou
un traitement de mise en solution à une température de 510 à 800°C pour faire baisser la température à laquelle la transformation austénitique se produit.

8.  Procédé selon l'une quelconque des revendications 1 à 7, dans lequel le traitement thermomécanlque (TMT) comprend l'électro-stimulation avec une densité de courant de 500 à 2000 A/cm$^2$.

9.  Procédé pour préparer un dispositif médical comprenant un alliage à mémoire de forme (SMA) mettant en application un double effet à mémoire de forme, comprenant l'étape conslstant à traiter un SMA selon le procédé tel que défini dans l'une quelconque des revendications 1 à 8.

10. Procédé selon la revendication 9, dans lequel ledit dispositif médical est un stent.

**11.** Procédé pour la fabrication d'un stent médical à partir d'un alliage de NiTi, qui est un fil présentant un premier diamètre, le stent ayant soit la forme d'un fil avec un second diamètre ou la forme d'une bande, le stent affichant un double effet à mémoire de forme (SME) ayant un état de mémoire austénitique et martensitique avec des formes austénitiques et martensitiques associées, respectivement, le procédé comprenant les étapes consistant à :

(a) chauffer un échantillon du fil de NiTi à une température de 450 à 550°C pendant 0,6 à 2,5 heures, et tester ensuite l'échantillon concernant une différence de température entre $A_s$ et $A_f$, dans laquelle $A_s$ est une température à laquelle la transformation austénitique, c'est-à-dire la transformation entre l'état martensitique et l'état austénitique, commence, et $A_f$ est une température à laquelle la transformation austénitique se termine ;
(b) soumettre le fil à un premier traitement thermique basé sur la différence A, - A, obtenue à l'étape (a), de la manière suivante :

lorsque la différence est inférieure à 7°C, traiter thermiquement le fil à la température de 450 à 500°C pendant 0,6 à 1,0 heure ;
lorsque la différence est supérieure à environ 7°C, traiter thermiquement le fil à une température de 610 à 550°C pendant 1,0 à 2,5 heures ;

(c) soumettre le fil à un traitement thermomécanique, comprenant le laminage à chaud du fil à une vitesse de déformation inférieure à 5 $sec^{-1}$, avec le chauffage interne simultané par électro-stimulation à une densité de courant de 500 à 2000 $A/cm^2$ d'une partie du fil à laquelle la déformation se produit, la déformation à cette étape étant inférieure à 55% ;
(d) lorsque la déformation à l'étape (c) ne produit pas une forme transversale de la forme finale, soumettre le fil à un traitement thermique intermédiaire à une température de 500 à 550°C, pendant 0,5 à 2 heures et répéter ensuite l'étape (c) ; et
(e) soumettre le fil à un traitement thermique final et à un traitement à mémoire qui comprend les étapes consistant à :

(i) enrouler le fil ou la bande obtenu à l'étape (c) sur un mandrin ayant un diamètre que le stent prend à l'état austénitique ;
(ii) soumettre le fil à un traitement thermique de polygonisation à 450 à 550°C pendant 0,6 à 1,5 heures, ensuite à un traitement de mise en solution à environ 600 à 800°C pendant 2 à 50 minutes, et ensuite à un traitement de vieillissement à 350 à 500°C pendant 0,15 à 2,5 heures ;
(iii) déformer le fil en l'enroulant sur un mandrin ayant un diamètre de conditionnement, la déformation étant inférieure à environ 7%, et étant réalisée à une température T qui satisfait la formule suivante :

$$T < M_s + 30°C$$

dans laquelle $M_s$ est une température à laquelle la transformation martensitique commence, et ensuite chauffer le fil ou la bande à une température de ou supérieure à celle à laquelle la transformation austénitique se termine ;
moyennant quoi on obtient un stent avec un état austénitique auquel il a un diamètre pris à l'étape (i) ci-dessus et un état martensitique auquel il a un diamètre qui est un diamètre intermédiaire entre le diamètre de conditionnement et le diamètre austénitique.

**12.** Procédé pour la fabrication d'un stent médical à partir d'un fil de NiTi ayant un premier diamètre, le stent ayant soit la forme d'un fil avec un second diamètre ou la forme d'une bande, le stent affichant un double effet à mémoire de forme (SME) ayant un état de mémoire austénitique et martensitique avec des formes austénitiques et martensitiques associées, respectivement, le procédé comprenant les étapes consistant à :

(a) chauffer un échantillon d'un fil en alliage à base de nIckel titane à une température de 450 à 550°C pendant 0,5 à 2,5 heures, et tester ensuite l'échantillon concernant la différence de température entre $A_s$ et $A_f$, dans laquelle $A_s$ est une température à laquelle la transformation austénitique, c'est-à-dire la transformation entre l'état martensitique et l'état austénitique, et $A_f$ est une température à laquelle la transformation austénitique se termine ;
(b) soumettre le fil à un premier traitement thermique basé sur la différence $A_s$ - $A_f$ obtenue à l'étape (a), de la manière suivante :

lorsque la différence est inférieure à envIron 7°C, traiter thermiquement le fil à une température de 450 à 500°C pendant 0,5 à 1,0 heure ;
lorsque la différence est supérieure à 7°C, traiter thermiquement le fil à une température de 510 à 550°C pendant 1,0 à 2,5 heures ;

(c) soumettre le fil à un traitement thermomécanique, comprenant le laminage à chaud du fil à une vitesse de déformation inférieure à 5 sec$^{-1}$, avec le chauffage interne simultané d'une partie du fil à laquelle la déformation se produit, par électro-stimulation à une densité de courant de 500 à 2000 A/cm$^2$, la déformation à cette étape étant inférieure à 55% ;

(d) lorsque la déformation à l'étape (c) ne produit pas de forme transversale de la forme finale, soumettre le fil à un traitement thermique intermédiaire à une température d'environ 600 à 550°C pendant 0,2 heure et répéter ensuite l'étape (c) ; et

(e) soumettre le fil à un traitement thermique final et à un traitement à mémoire, qui comprend les étapes consistant à :

> (i) enrouler la fil ou la bande obtenu à l'étape (c) sur un mandrin ayant un diamètre de conditionnement qui est différent du diamètre que le stent prend à l'état austénitique ;
>
> (ii) soumettre le fil à un traitement thermique à 450 à 500°C pendant 0,5 à 2 heures, ensuite à une polygonisation et un traitement de mise en solution à 600 à 800°C pendant 2 à 50 minutes, et ensuite à un traitement de vieillissement à 350 à 500°C pendant 0 à 2 heures ;
>
> (iii) enrouler le fil ou la bande sur un mandrin ayant un diamètre que le stent prend à l'état austénitique ;
>
> (iv) soumettre le fil à un traitement thermique à mémoire à environ 500 à 600°C pendant plus de 10 minutes, et ensuite au traitement de vieillissement à 350 à 600°C pendant 0,15 à 2,15 heures ;

moyennant quoi on obtient un stent ayant un état austénitique avec un diamètre dans lequel le fil est formé à l'étape (iii), et un état martensitique dans lequel le stent a un diamètre qui est un diamètre intermédiaire entre le diamètre de conditionnement et le diamètre du stent à l'état austénitique.

13. Procédé pour la fabrication de l'implant de la racine de la dent pour l'alliage NiTi, affichant un double effet à mémoire de forme ayant un état de mémoire austénitique et martensitique avec les formes austénitiques et martensitiques associées, respectivement, le procédé comprenant les étapes consistant à :

> (a) chauffer un échantillon d'une tige en NiTi à une température de 450 à 550°C pendant 0,5 à 2,5 heures, et tester ensuite l'échantillon concernant la différence de température entre $A_s$

et $A_f$, dans laquelle $A_s$ est une température à laquelle la transformation austénitique, c'est-à-dire la transformation entre l'état martensitique et l'état austénitique commence, et $A_f$ est une température à laquelle la transformation austénitique se termine ;

(b) soumettre la tige à un premier traitement thermique basé sur la différence $A_f$ - $A_s$ obtenue à l'étape (a), de la manière suivante :

> lorsque la différence est infiérieure à 7°C, traiter thermiquement la tige à une température de 450 à 500°C pendant 0,5 à 1,0 heure :
>
> lorsque la différence est supérieure à 7°C, traiter thermiquement la tige à une température de 610 à 550°C pendant 1,0 à 2,5 heures ;

(c) soumettre la tige à un traitement thermomécanique comprenant l'étirage à chaud avec une vitesse de déformation inférieure à 6 sec$^{-1}$ en chauffant simultanément, la déformation totale à cette étape étant inférieure à 55% ;

(d) lorsque la déformation à l'étape (c) ne produit pas une forme transversale de la forme finale, soumettre la tige au traitement thermique intermédiaire à une température de 600 à 550°C pendant 0,5 à 2 heures et répéter ensuite l'étape (c) ;

(e) usiner la tige pour produlre la forme de l'implant ;

(f) soumettre l'Implant au traitement thermique final et à un traitement à mémoire qui comprend les étapes consistant à :

> (l) étendre les segments de force de l'implant à un diamètre que l'implant prend à l'état austénitique,
>
> (ii) soumettre l'implant à un traitement thermique de polygonisation à 450 à 550°C pendant 0,5 à 1,5 heures, ensuite à un traitement de mise en solution à 600 à 800°C pendant 2 à 50 minutes, et ensuite à un traitement de vieillissement à environ 350 à 500°C pendant 0,15 à 2,5 heures ;
>
> (iii) déformer les segments de force de l'implant à un diamètre de conditionnement avec une déformation inférieure à 7% et étant réalisée à une température T < $M_s$ + 30°C, dans laquelle $M_s$ est une température à laquelle la transformation martensitique commence et chauffer ensuite l'implant à une température de ou au dessus de celle à laquelle la transformation austénitique se termine ;

moyennant quol on obtient l'implant avec un état austénitique auquel il a un diamètre pris à l'étape (i) ci-dessus, et un état martensitique auquel il a un diamètre qui est un diamètre intermédiaire entre le diamètre de conditionnement et le diamètre austénitique.

14. Procédé pour la fabrication d'un couplage de tuyau à partir d'un alliage de NiTi, affichant un double effet à mémoire de forme ayant des états de mémoire austénitique et martensitique avec les formes austénitiques et martensitiques associées, respectivement, le procédé comprenant les étapes consistant à :

(a) chauffer un échantillon de la tige de NiTi à une température de 450 à 550°C pendant 0,5 à 2,5 heures, et tester ensuite l'échantillon concernant la différence de température entre $A_s$ et $A_f$, dans laquelle $A_s$ est une température à laquelle la transformation austénitique, c'est-à-dire la transformation entre l'état martensitique et l'état austénitique, commence, et $A_f$ est une température à laquelle la transformation austénitique se termine ;

(b) soumettre la tige à un premier traitement thermique basé sur la différence $A_f$ - $A_s$ obtenue à l'étape (a), de la manière suivante :

lorsque la différence est inférieure à environ 7°C, traiter thermiquement la tige à une température de 460 à 500°C pendant 0,5 à 2,0 heures ;
lorsque la différence est supérieure à 7°C, traiter thermiquement le fil à une température de 510 à 550°C pendant 1,0 à 2,5 heures ;

(c) soumettre la tige au traitement thermomécanique comprenant l'étirage à chaud avec une vitesse de déformation inférieure à 5 sec$^{-1}$ en chauffant simultanément, la déformation totale étant inférieure à 55% ;

(d) lorsque la déformation à l'étape (c) ne produit pas de forme transversale de la forme finale, soumettre la tige à un traitement thermique intermédiaire à une température de 500 à 550°C, pendant 0,5 à 2 heures et répéter ensuite l'étape (c) ; et

(e) usiner la tige pour produire le couplage d'un cylindre creux avec un diamètre interne de conditionnement ;

(f) soumettre le cylindre au traitement thermique final et à un traitement à mémoire, qui comprend les étapes consistant à :

(i) soumettre le cylindre à un traitement thermique de polygonisation à 450 à 550°C pendant environ 0,5 à 1,5 heures, ensuite au traitement de mise en solution à 600 à 800°C pendant 2 à 50 minutes et ensuite à un traitement de vieillissement à 350 à 500°C pendant 0 à 2,5 heures ;
(ii) étendre le cylindre à un diamètre que le couplage prend à l'état austénitique ;
(iii) soumettre le cylindre à un traitement thermique à mémoire à 500 à 600°C pendant plus de 10 minutes, et ensuite à un traitement de vieillissement à 350 à 500°C pendant 0,15 à 2,5 heures ;

moyennant quoi on obtient un couplage ayant un état austénitique avec un diamètre dans lequel il a été formé à l'étape (ii) et un état martensitique dans lequel le couplage a un diamètre qui est un diamètre intermédiaire entre le diamètre interne de conditionnement et le diamètre de couplage à l'état austénitique.

FIG.1